(19)

**Europäisches Patentamt**
**European Patent Office**
**Office européen des brevets**

(11) **EP 4 699 534 A1**

(12) **EUROPEAN PATENT APPLICATION**

(43) Date of publication:
25.02.2026 Bulletin 2026/09

(21) Application number: 25197423.4

(22) Date of filing: 21.08.2025

(51) International Patent Classification (IPC):
*A61B 5/145* (2006.01) *A61B 5/1455* (2006.01)

(52) Cooperative Patent Classification (CPC):
**A61B 5/14532; A61B 5/1455**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 21.08.2024 TW 113131401
22.08.2024 US 202418812891

(71) Applicant: **CW Medtech Limited.**
**Taipei City (TW)**

(72) Inventor: **LEE, PO-LEI**
**32001 TAOYUAN CITY (TW)**

(74) Representative: **Lavoix**
**Bayerstraße 83**
**80335 München (DE)**

(54) **APPARATUS AND METHOD FOR NON-INVASIVE MEASUREMENT OF BLOOD GLUCOSE LEVEL**

(57) An apparatus and a method for non-invasive measurement of blood glucose level are provided, which are capable of driving two or more light sources to emit lights in different wavelength bands toward a part of a body of a user (e.g., a subject), deriving amplitude values from fiducial points in at least one pulse of a respective PPG signal corresponding to each wavelength band, and estimating the blood glucose level of the user using a plurality of amplitude ratios derived from the amplitude values corresponding to different combinations of wavelength bands. The lights emitted by the two or more light sources are in different wavelength bands, indicating that the lights have different characteristics of absorption coefficients for at least glucose and hemoglobin. This helps build the regression model using more data points collected for cross-referencing and calibration compared to using one light source, thereby improving overall accuracy in the estimation of the blood glucose level of the subject.

EP 4 699 534 A1

**Description**

**TECHNICAL FIELD**

The present disclosure relates to a health data sensor, and more particularly, to an apparatus and a method for non-invasive measurement of blood glucose level.

**BACKGROUND**

Measurement of blood glucose are categorized into invasive and non-invasive types. Invasive blood glucose meters are relatively mature and accurate but painful to the subject because they require pricking the skin to obtain a blood sample. The risk of infection is particularly heightened with frequent measurements. Additionally, each measurement requires a certain amount of time and operational steps, significantly increasing the cost for subjects who need frequent monitoring.

Non-invasive blood glucose meters, which do not require pricking the skin, can be implemented through optical measurement, electromagnetic field measurement, ultrasound measurement, and skin impedance measurement. Non-invasive blood glucose meters are easy to operate, but individual differences between users, along with technical differences in data analysis across different brands, leading to measurement errors in blood glucose level and affect data accuracy. As a result, their precision is generally lower than that of invasive blood glucose meters. This is especially true at the extreme ends of the glucose measurement range, where frequent calibration is needed to ensure data accuracy. Furthermore, the measurement results may be affected by external environmental factors, such as temperature, humidity, light, etc. Therefore, it is necessary to develop more effective technologies to improve the accuracy and environmental sensitivity of non-invasive blood glucose meters.

**SUMMARY**

One of the objects of the present disclosure is to provide an improved manner for non-invasive measurement of a subject's blood glucose level. The improvement may lie in accuracy, ease of use, or both.

In an aspect of the present disclosure, an apparatus for non-invasive measurement of a blood glucose level of a subject is provided. The apparatus includes a first light source, a second light source, a sensor, and a processor. The first light source is configured to emit first light in a first wavelength band toward a part of a body of the subject. The second light source is configured to emit second light in a second wavelength band toward the part of the body. The sensor is configured to, in response to receiving the first light and the second light having been reflected by or passed through the part of the body, respectively output a first photoplethysmography (PPG) signal and a second PPG signal, each of the first PPG signal and the second PPG signal comprising at least one pulse. The processor is configured to: identify, from the at least one pulse of the first PPG signal, a first set of fiducial points and a first set of amplitude value s associated with the first set of fiducial points; identify, from the at least one pulse of the second PPG signal, a second set of fiducial points and a second set of amplitude values associated with the second set of fiducial points; deriving a plurality of amplitude ratios from the first and second sets of amplitude values; and estimating, based on the derived plurality of amplitude ratios, the blood glucose level of the subject. Glucose has lower absorption level in the first wavelength band than the second wavelength band. Hemoglobin has higher absorption level in the first wavelength band than in the second wavelength band.

The relative difference in the absorption level of glucose between the first and second wavelength bands makes the second light more sensitive to the glucose level in the blood of the blood vessels in the part of the body of the subject. This sensitivity contributes to the measurement of the amount of glucose in the blood. Also, the relative difference in the absorption level of hemoglobin makes the first light more sensitive to the amount of hemoglobin in the blood. Since the hemoglobin amount can be a good indicator of the volume of the blood that affects the lights, the first light is more sensitive to the blood volume, contributing to its measurement. As blood glucose level is affected by both the amount of glucose in the blood and the volume of the blood being measured, the ability to measure both improves the accuracy of measuring the blood glucose level of the subject with one light that is only sensitive to glucose.

Also, ratio is a useful way to combine the information available in two constituting numbers. Therefore, using the amplitude ratios from the sets of amplitude values identified from the pulses of respective PPG signals that result from lights in the first and second wavelength bands helps simplify the estimation calculation while preserving the information available from the two lights. Some comparative studies have relied solely on a single glucose-related wavelength to measure blood glucose levels, conducting quantitative spectral analysis based on fixed blood volume glucose concentrations. However, this single-wavelength measurement approach cannot account for individual differences in clinical settings. Moreover, due to the variability in blood volume within blood vessels, it is unable to calibrate the glucose absorbance in the illuminated region, resulting in reduced measurement accuracy. In contrast, the present disclosure utilizes amplitude ratios that have information from both the first and second wavelength bands, thereby improving measurement accuracy through, e.g., better accounting for the variability in blood volume within blood vessels.

Another advantage of using two light sources with different wavelength bands is that more data points may be collected for cross-calibration than using one light source, thereby improving overall accuracy in the estimation of the blood glucose level of the subject.

Optionally, the first set of fiducial points may comprise a first valley, a first systolic peak, a first dicrotic notch and a first diastolic peak. Optionally, the second set of fiducial points may comprise a second valley, a second systolic peak, a second dicrotic notch and a second diastolic peak. These fiducial points are related to cardiac cycles and thus provide information on the cardiovascular system of the subject, enhancing the accuracy of blood glucose level estimation. Also, the use of consistently defined fiducial points across different measurement samples can improve the stability of measurement accuracy.

Optionally, the first set of amplitude values may comprise a first systolic peak amplitude, a first dicrotic notch amplitude, and a first diastolic peak amplitude each measured with reference to a first valley amplitude. Optionally, the second set of amplitude values may comprise a second systolic peak amplitude, a second dicrotic notch amplitude, and a second diastolic peak amplitude each measured with reference with a second valley amplitude.

Optionally, the plurality of amplitude ratios may comprise at least one of: the second systolic peak amplitude divided by the first systolic peak amplitude; the second dicrotic notch amplitude divided by the first dicrotic notch amplitude; and the second diastolic peak amplitude divided by the first diastolic peak amplitude. The flexibility in the selection of amplitude ratios to be included in the estimation process helps improve the optimal balance between calculation complexity and measurement accuracy.

Optionally, the plurality of amplitude ratios may comprise at least one of: the logarithm of the second systolic peak amplitude divided by the logarithm of the first systolic peak amplitude; the logarithm of the second dicrotic notch amplitude divided by the logarithm of the first dicrotic notch amplitude; and the logarithm of the second diastolic peak amplitude divided by the logarithm of the first diastolic peak amplitude. As logarithmic scales reduce wide-ranging quantities to smaller scopes, the inclusion of the logarithm of some or all of the amplitudes may help the processor better handle widely varied amplitude values. Optionally, any one of the logarithm ratios may be subtracted by a constant.

Optionally, the processor may be further configured to apply the plurality of amplitude ratios into a regression model. Optionally, estimating the blood glucose level of the subject may be further based on the regression model.

Optionally, the absorption level of glucose in the first wavelength band may be less than 0.003 $cm^{-1} \cdot (mol/L)^{-1}$. With sufficiently low level of glucose absorption, the information from the first light and therefore the first PPG signal may be more representative of the blood volume, thereby improving accuracy of estimation of the blood glucose level of the subject. Unless otherwise indicated in the present disclosure, $L$ denotes liter ($cm^3$) and $mol$ is the unit of mole.

Optionally, the absorption levels for both oxyhemoglobin (HbO2) and deoxyhemoglobin (Hb) in the first wavelength band may be more than 10 $cm^{-1} \cdot (mol/L)^{-1}$. Since the first wavelength band is chosen to make the first light sensitive to both HbO2 and Hb, the first light is sensitive to blood carrying different levels of oxygen, making the information therefrom more representative of the blood volume, thereby improving the accuracy of blood glucose level estimation.

Optionally, melanin may have higher absorption level in the first wavelength band than in the second wavelength band. The sensitivity of the first light to melanin also makes it more suitable for retrieving information regarding blood volume.

Optionally, HbO2 and Hb may have substantially identical absorption level in the first wavelength band. Optionally, the absorbance coefficient for both Hb and HbO2 may be around 10 $cm^{-1} \cdot (mol/L)^{-1}$.

Optionally, the first wavelength band may be green light. Optionally, the first light may have a wavelength of around 520nm. Green light emitting devices (including those that emit wavelengths around 520 nm wavelength) are widely available for integration into wearable devices, making the technology easier to implement and become readily available.

Optionally, the absorption level of glucose in the second wavelength band may be more than 0.005 $cm^{-1} \cdot (mol/L)^{-1}$. With sufficiently high level of glucose absorption, the information from the second light and therefore the second PPG signal may be more representative of the amount of glucose, thereby improving accuracy of estimation of the blood glucose level of the subject.

Optionally, the absorption levels of hemoglobin in the second wavelength band for both Hb and HbO2 may be less than 10 $cm^{-1} \cdot (mol/L)^{-1}$. Since the second wavelength band is chosen to make the second light sufficiently insensitive to both HbO2 and Hb, information from the second light may be less affected by blood volume and more representative of glucose, thereby improving the accuracy of blood glucose level estimation.

Optionally, the second wavelength band may be near-infrared (NIR) light. Optionally, the second light may have a wavelength of around 940nm. Light-emitting devices in this band and around this wavelength are widely available, making the technology easier to implement and become readily available.

Optionally, the apparatus may further comprise a third light source configured to emit third light in a third wavelength band towards the part of the body of the subject. Optionally, the absorption level of hemoglobin in the third wavelength band may be greater than 0.2 $cm^{-1} \cdot (mol/L)^{-1}$.

Optionally, glucose has higher absorption level in the third wavelength band than the second wavelength band, optionally the absorption level of glucose in the third wavelength band is greater than 0.2 $cm^{-1} \cdot (mol/L)^{-1}$. Providing a third light that is also sensitive to glucose helps further improving the accuracy of the estimation of blood glucose level in the subject.

Optionally, the sensor may be further configured to, in response to receiving the third light having been reflected by or passed through the part of the body, output a third PPG signal, the third PPG signal comprising at least one pulse. Optionally, the processor may be further configured to identify, from the at least one pulse of the third PPG signal, a third set of fiducial points and a third set of amplitude values associated with the third set of fiducial points. Optionally, the third set of fiducial points may comprise a third valley, a third systolic peak, a third dicrotic notch and a third diastolic peak. Optionally, the plurality of amplitude ratios may be further derived from the third set of amplitude values. Optionally, the third set of amplitude values may comprise a third systolic peak amplitude, a third dicrotic notch amplitude and a third diastolic peak amplitude each measured with reference to a third valley amplitude.

Optionally, the plurality of amplitude ratios may further comprise at least one of: the third systolic peak amplitude divided by the first systolic peak amplitude; the third dicrotic notch amplitude divided by the first dicrotic notch amplitude; the third diastolic peak amplitude divided by the first diastolic peak amplitude; the logarithm of the third systolic peak amplitude divided by the logarithm of the first systolic peak amplitude; the logarithm of the third dicrotic notch amplitude divided by the logarithm of the first dicrotic notch amplitude; and the logarithm of the third diastolic peak amplitude divided by the logarithm of the first diastolic peak amplitude. Optionally, any one of the logarithm ratios may be subtracted by a constant.

Optionally, the third wavelength band may be infrared light. Optionally, the third light may have a wavelength of around 1550 nm. The wavelength around 1550 nm exhibits high sensitivity to glucose and therefore can further improve the accuracy of measuring blood glucose. Since three light sources with different wavelength bands exhibit different combinations of characteristics or sensitivity to glucose and hemoglobin, an advantage of such design is that more data points may be collected for cross-calibration in a more robust manner, compared to using one light source or two light sources. Additionally, the amplitude ratios that have information from all the first to third wavelength bands can be utilized by the regression model to improve measurement accuracy of the blood glucose level of the user in a non-invasive manner.

Optionally, the first light source and the second light source may be positioned on a same side or opposite sides of the part of the body of the subject. This flexibility may improve the ease of use of the apparatus of the present disclosure.

In another aspect of the present disclosure, a method for estimating a blood glucose level of a subject using non-invasive measurement is provided. The method comprises: driving a first light source and a second light source to emit first light in a first wavelength band and second light in a second wavelength band toward a part of a body of a subject, respectively; in response to receiving the first light and the second light having been reflected by or passed through the part of the body, respectively outputting a first photoplethysmography (PPG) signal and a second PPG signal via a sensor, wherein each of the first PPG signal and the second PPG signal comprises at least one pulse; identifying, from the at least one pulse of the first PPG signal, a first set of fiducial points and a first set of amplitude value s associated with the first set of fiducial points; identifying, from the at least one pulse of the second PPG signal, a second set of fiducial points and a second set of amplitude values associated with the second set of fiducial points; deriving a plurality of amplitude ratios from the first and second sets of amplitude values; and estimating, based on the derived plurality of amplitude ratios, the blood glucose level of the subject. Glucose has lower absorption level in the first wavelength band than the second wavelength band. Hemoglobin has higher absorption level in the first wavelength band than in the second wavelength band.

Any of the optional features mentioned above for the apparatuses of the present disclosure may also be optional features to the methods of the present disclosure.

## BRIEF DESCRIPTION OF THE DRAWINGS

Aspects of the present disclosure are best understood from the following detailed description when read with the accompanying figures. It is noted that in accordance with the standard practice in the industry, various features are not drawn to scale. In fact, the dimensions of the various features may be arbitrarily increased or reduced for clarity of discussion.

FIG. 1 is a block diagram of an apparatus for non-invasive measurement of blood glucose level in accordance with an embodiment of the present disclosure.

FIG. 2 is a diagram illustrating exemplary usage scenarios of a wearable device including the apparatus in FIG. 1.

FIG. 3 is a waveform diagram illustrating various PPG signals detected from lights in different wavelength bands in accordance with an embodiment of the present disclosure.

FIG. 4 is a waveform diagram of a single PPG signal in FIG. 3.

FIG. 5 is a flowchart of a method for non-invasive measurement of blood glucose level in accordance with an embodiment of the present disclosure.

Corresponding numerals and symbols in the different figures generally refer to corresponding parts unless otherwise indicated. The figures are drawn to clearly illustrate the relevant aspects of the various embodiments and are not necessarily drawn to scale.

## DETAILED DESCRIPTION

In order to make the above and other objectives, features, and advantages of the present disclosure more obvious and understandable, the following exemplifies the preferred embodiments of the present disclosure, combined with the accompanying drawings, and describe in detail as follows.

It should be understood that the following detailed description of the embodiments is merely illustrative and should not be construed as limiting the disclosure as defined by the claims. In general, unless the following embodiments explicitly define the terms used, the terms included in the disclosure should not be interpreted as limiting the techniques described herein to the specific embodiments disclosed in this specification. Accordingly, the actual scope of the techniques described herein should encompass the disclosed embodiments as well as all equivalents for implementing these techniques.

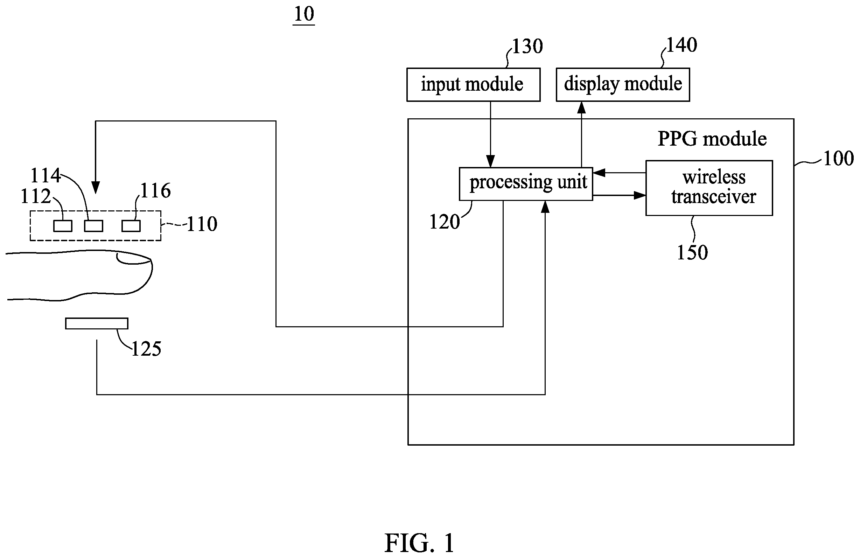

FIG. 1 is a block diagram of an apparatus for non-invasive measurement of blood glucose level in accordance with an embodiment of the present disclosure.

In an embodiment, an apparatus 10 is a wearable device configured to perform non-invasive measurement of blood glucose level of a part of a body of a user (such as a subject), which can include at least one of fingers, arms, legs, ears, head, face, neck, torso, wrist, feet, among others. The apparatus 10 includes a PPG module 100, a light-emitting module 110, an input module 130, and a display module 140, as depicted in FIG. 1.

The input module 130 includes one or more peripheral devices, such as a keyboard, a touch panel, physical buttons, and the like, on which the user can operate the apparatus 10. The display module 140 may be a liquid crystal display (LCD) module, an organic light-emitting diode (OLED) display, or the like. The display module 140 is configured to display operating information of the apparatus 10 and/or blood glucose information measured by the apparatus 10, but the present disclosure is not limited thereto.

In an embodiment, the PPG module 100 includes a processing unit 120 and a wireless transceiver 150. The processing unit 120 may include a microprocessor, a processor, an application-specific integrated circuit (ASIC), a field programmable gate array (FPGA), or similar processing components capable of processing data and/or executing algorithms and various calculations as described below. The wireless transceiver 150 may include a Bluetooth transceiver, a Wi-Fi transceiver, or other transceivers compatible with wireless standards, such as cellular communication, RFID and short-range communication, among others. Accordingly, the apparatus 10 can be in communicative connection with a mobile device, a personal computer, a cloud server through the wireless transceiver 150.

In an embodiment, the light-emitting module 110 includes at least light sources 112 and 114. In response to a control signal from the processing unit 120, the light sources 112 and 114 emit first light in a first wavelength band and second light in a second wavelength band toward a part of the body of the user. Additionally, glucose has a relatively lower absorption level (e.g., lower absorption coefficient in glucose) in the first wavelength band than the second wavelength band, while hemoglobin (including oxyhemoglobin (HbO2) and deoxyhemoglobin (Hb)) has a relatively higher absorption level (e.g., higher absorption coefficient in hemoglobin) in the first wavelength band than the second wavelength band.

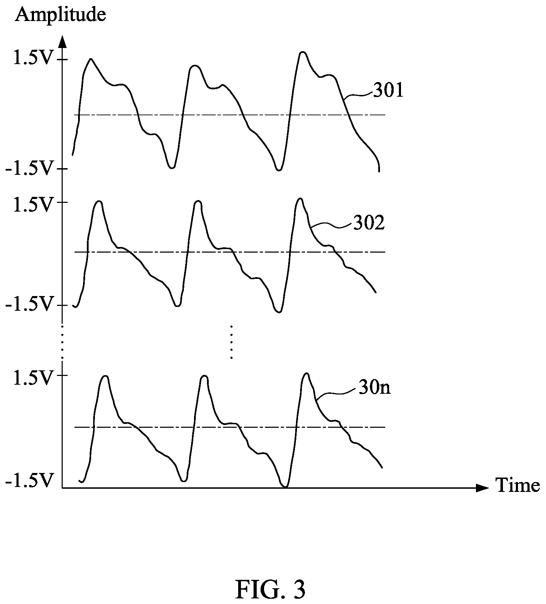

In a first arrangement of two light sources, for illustrative purposes, the light sources 112 and 114 are disposed on a first side (e.g., top side) of a part (e.g., a finger) of the body of the user, while the light sensor 125 is disposed on a second side (e.g., bottom side) opposite to the first side of the part of the body of the user, as depicted in FIG. 1. Accordingly, the light sensor 125 can receive the first light and the second light passing through the part of the body of the user, thereby generating a first PPG signal and a second PPG signal, respectively. The first PPG signal and the second PPG signal may each include at least one pulse, as illustrated by curve 301 and 302 in FIG. 3, respectively.

In a second arrangement of two light sources, the light sources 112 and 114, and the light sensor 125 are disposed on the same side of a part of the body of the user. Accordingly, the light sensor 125 can receive the first light and the second light reflected by the part of the body of the user, thereby generating a first PPG signal and a second PPG signal, respectively. The first PPG signal and the second PPG signal may each include at least one pulse, as illustrated by curve 301 and 302 in FIG. 3, respectively.

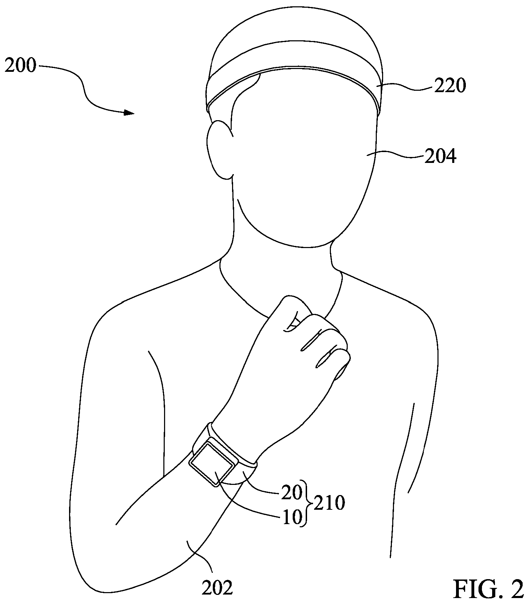

FIG. 2 is a diagram illustrating exemplary usage scenarios of a wearable device including the apparatus in FIG. 1. Please refer to both FIGS. 1 and 2.

In an embodiment, the apparatus 10 can be combined with different types of wearable component (e.g., watch strap, wristband, head band, eye frame, ring frame, bracelet, necklace, among others) to form wearable devices of different types, such as a smart watch, smart wristband, smart head band, smart glasses, smart ring, smart bracelet, smart necklace, among others. When the user wears a wearable device including the apparatus 10, the wearable device is capable of measuring the blood glucose level of the user in a non-invasive manner using two or more light sources.

For illustrative purposes, the wearable device 210 shown in FIG. 2 may be a smart watch or a smart wristband, which combines the apparatus 10 shown in FIG. 1 with a wearable component 20, such as a watch strap or a wristband. The user 200 can wear the wearable device 210 on the wrist of his or her arm 202, allowing the apparatus 10 to measure the glucose level of the user 200 from the wrist of arm 202 in a non-invasive manner. In an embodiment, the light sources 112 and 114 and the light sensor 125 of the apparatus 10 are on the same side, enabling the light sensor 125 to receive lights reflected by a part of the body of the user. In other embodiments, some of the components of the apparatus 10 may reside on the wearable component 20. For example, the light sensor 125 may reside on the wearable component 20 so that the lights may have passed through a part of the body of the user before being received by the sensor 125. In yet other embodiments, other components of the apparatus 10 may be located on the wearable component 20, which may provide more flexibility to designing and manufacturing the wearable device 210. For example, distributing some of the processing unit 120, input module 130, display module 140 and wireless transceiver 150 to the wearable component 20 may leave more space to the display module 140 and the battery (not shown) of the apparatus 10 and/or help prevent too much weight of the wearable device 210 from concentrating on one side of it. Also, as the technology for curved display becomes more popularized, the wearable component 20 may also include some display component/function. In other embodiments, both the light sources 112, 114, and 116 and the light sensor 125 can be located on the wearable component 20, enabling the light sensor 125 to collect lights reflected by or passing through the body of the user in different directions (e.g., directions more parallel to the direction of the blood vessels, directions more perpendicular thereto, and directions that form an angle within relevant angular range(s) with one or more particular blood vessels). The ability to control the direction of the light received by the sensor may help improve the accuracy of the estimation of the level of blood glucose or other biological/physiological indicators by aligning the light(s) with directions (e.g., along a major blood vessel) that contain more relevant information.

In another embodiment, the apparatus 10 can be combined with a head band 220 to form a wearable device of a different type, allowing the user 200 to wear the wearable device on his or her head 204 via the head band 220. Accordingly, the wearable device can measure the glucose level of the user 200 from the face or forehead of the user 200 in a non-invasive manner. The ability to collect physiological information from a body part different from the wrist may improve user convenience; in some cases, the information from different body parts may complement each other, leading to improved overall accuracy.

**First Scenario using Two Light Sources**

Turning back to FIG. 1, in a first scenario of two light sources, such as the first light source 112 and the second light source 114, the first wavelength band corresponds to green light, optionally around 520 nm. The second wavelength band corresponds to near-infrared (NIR) light, optionally around 940 nm. Alternatively, the first wavelength band corresponds to green light, optionally around 530 nm. The second wavelength band corresponds to near-infrared (NIR) light, optionally around 830 nm. The two-light-source scenario may also be called a "two-channel" or "two-source" scenario.

In the first scenario, the absorption level (e.g., molar absorption coefficient, or "absorption coefficient" in short) of glucose in the first wavelength band is less than $0.003\ cm^{-1} \cdot (mol/L)^{-1}$, and/or the absorption levels for both HbO2 and Hb in the first wavelength band are more than $10\ cm^{-1} \cdot (mol/L)^{-1}$. Additionally, melanin has a relatively higher absorption level (e.g., absorption coefficient) in the first wavelength band than in the second wavelength band. Furthermore, HbO2 and Hb have substantially identical absorption level in the first wavelength band, optionally the absorption coefficients for both Hb and HbO2 are around $10\ cm^{-1} \cdot (mol/L)^{-1}$. The absorption level of glucose in the second wavelength band is more than $0.005\ cm^{-1} \cdot (mol/L)^{-1}$, and/or the absorption levels of hemoglobin in the second wavelength band for both Hb and HbO2 are less than $10\ cm^{-1} \cdot (mol/L)^{-1}$.

**Second Scenario Using Two Light Sources**

In a second scenario using two light sources, the first wavelength band corresponds to green light, optionally around 520 nm. The second wavelength band corresponds to near-infrared (NIR) light or short-wave infrared (SWIR) light, optionally around 1550 nm.

In the second scenario, the absorption level of glucose in the first wavelength band is less than $0.003\ cm^{-1} \cdot (mol/L)^{-1}$, and/or the absorption levels for both HbO2 and Hb in the first wavelength band are more than $10\ cm^{-1} \cdot (mol/L)^{-1}$. Additionally, melanin has a relatively higher absorption level (e.g., absorption coefficient) in the first wavelength band than in the second wavelength band. Furthermore, HbO2 and Hb have substantially identical absorption level in the first wavelength band, optionally the absorption coefficients for both Hb and HbO2 are around $10\ cm^{-1} \cdot (mol/L)^{-1}$. The

absorption level of glucose in the second wavelength band is higher than 0.2 $cm^{-1} \cdot (mol/L)^{-1}$, and/or the absorption levels of hemoglobin in the second wavelength band for both Hb and HbO2 are less than 0.2 $cm^{-1} \cdot (mol/L)^{-1}$.

**Scenario Using Three Light Sources**

In an embodiment, in addition to the light sources 112 and 114, the light-emitting module 110 includes a light source 116 configured to emit third light in a third wavelength band toward the part of the body in response to the control signal from the processor unit 120. In an embodiment, the first wavelength band corresponds to green light, optionally around 520 nm. The second wavelength band corresponds to near-infrared (NIR) light, optionally around 940 nm. The third wavelength band corresponds to near-infrared (NIR) light or short-wave infrared (SWIR) light, optionally around 1550 nm. In an alternative embodiment, the first wavelength band corresponds to green light, optionally around 530 nm. The second wavelength band corresponds to red light, optionally around 780 nm. The third wavelength band corresponds to near-infrared (NIR) light, optionally around 830 nm.

In a first arrangement of three light sources, the light sources 112, 114, and 116 are positioned on a first side of a part of the body of the user, while the light sensor 125 is positioned on a second side opposite to the first side of the part of the body of the user. Accordingly, the light sensor 125 can receive the first light, the second light, and the third light passing through the part of the body of the user, thereby generating a first PPG signal, a second PPG signal, and a third PPG signal, respectively. The first PPG signal, the second PPG signal, and the third PPG signal may each include at least one pulse. FIG. 1 provides an exemplary illustration of this arrangement.

In a second arrangement of three light sources, the light sources 112, 114, and 116 and the light sensor 125 are positioned on the same side of a part of the body of the user. Accordingly, the light sensor 125 can receive the first light, the second light, and the third light reflected by the part of the body of the user, thereby generating a first PPG signal, a second PPG signal, and a third PPG signal, respectively. The first PPG signal, the second PPG signal, and the third PPG signal may each include at least one pulse.

In the scenario using three light sources, the absorption level of glucose in the first wavelength band is less than 0.003 $cm^{-1} \cdot (mol/L)^{-1}$, and/or the absorption levels for both oxyhemoglobin (HbO2) and deoxyhemoglobin (Hb) in the first wavelength band are more than 10 $cm^{-1} \cdot (mol/L)^{-1}$. Additionally, melanin has a relatively higher absorption level in the wavelength band than in the second wavelength band. Furthermore, HbO2 and Hb have substantially identical absorption level in the first wavelength band, optionally the absorption coefficients for both Hb and HbO2 are around 10 $cm^{-1} \cdot (mol/L)^{-1}$. The absorption level of glucose in the second wavelength band is more than 0.005 $cm^{-1} \cdot (mol/L)^{-1}$, and/or the absorption levels of hemoglobin in the second wavelength band for both Hb and HbO2 are less than 10 $cm^{-1} \cdot (mol/L)^{-1}$. The absorption level of glucose in the third wavelength band is higher than 0.2 $cm^{-1} \cdot (mol/L)^{-1}$, and/or the absorption levels of hemoglobin in the third wavelength band for both Hb and HbO2 are less than 0.2 $cm^{-1} \cdot (mol/L)^{-1}$.

**Scenario Using More Than Three Light Sources**

In an embodiment, in addition to the light sources 112, 114, and 116, the light-emitting module 110 further includes more light sources configured to emit different lights in respective wavelength bands toward the part of the body in response to the control signal from the processor unit 120. In other words, N light sources can be disposed in the apparatus 10, where N > 3. The possible configurations of more than three light sources can be adapted from those described in the above embodiments related to the scenario using two and three light sources. The wavelengths or wavelength bands of other light sources other than the light sources 112, 114, and 116 can be designed or selected based on the characteristics of absorption coefficients to compounds/compositions of interest, such as glucose, hemoglobin, melanin, and the like.

In an arrangement, all N light sources of the apparatus 10 are disposed on the same side of a part of the body of the user as the side on which the light sensor 125 is disposed, enabling the light sensor 125 to receive respective lights emitted by the light sources and reflected by the part of the body, thereby generating N respective PPG signals, as exemplarily shown by curves 301 to 30N in FIG. 3. In an alternative arrangement, all N light sources of the apparatus 10 are disposed on a first side of a part of the body of the user, while the light sensor 125 is disposed on a second side opposite to the first side of the part of the body. In other alternative arrangements, all N lights sources are disposed on the first side, and more than one light sensors 125 may exist, one disposed on the first side and the other disposed on the second side. The light sensor on the first side may be configured to receive lights reflected by a body part of the subject, and that on the second side may be configured to receive lights having passed through a body part of the subject. In FIG 3, the bounds of the amplitude of the curves 301 to 30N are shown as 1.5V and minus 1.5V, but these numbers are just exemplary.

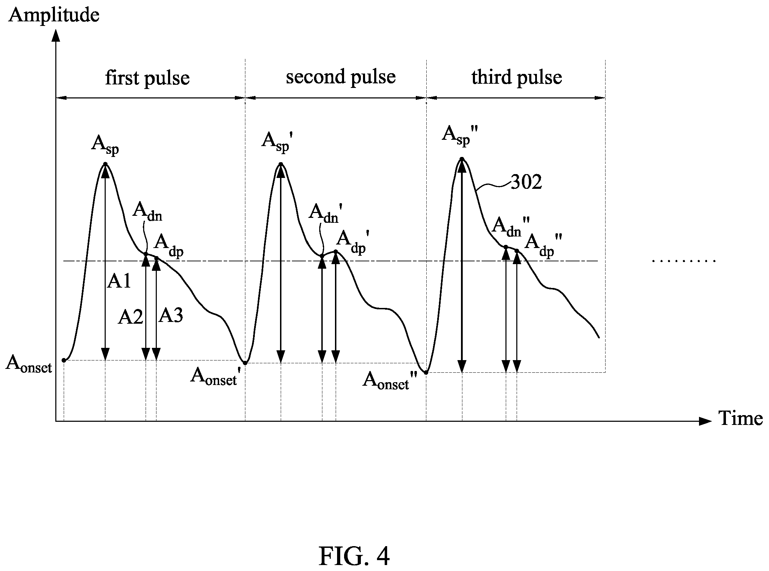

FIG. 4 is a waveform diagram of a single PPG signal in FIG. 3.

In an embodiment, for purposes of description, curve 302 in FIG. 3, which corresponds to the second PPG signal, is illustrated in FIG. 4. Three pulses of the second PPG signal, including a first pulse, a second pulse and a third pulse, are shown, each pulse including respective feature points or fiducial points. Examples of the feature points or fiducial points include onset (or valley), systolic peak, dicrotic notch, and diastolic peak. As illustrated in FIG. 4, the amplitudes of the onset, systolic peak (e.g., abbreviated as "sp"), dicrotic notch (e.g., abbreviated as "dn"), and diastolic peak (e.g., abbreviated as "dp") within the first pulse are denoted as $A_{onset}$, $A_{sp}$, $A_{dn}$, and $A_{dp}$, respectively. The amplitudes of the onset, systolic peak, dicrotic notch, and diastolic peak within the second pulse are denoted as $A_{onset}'$, $A_{sp}'$, $A_{dn}'$, and $A_{dp}'$, respectively (i.e., with the addition of one prime symbol). The amplitudes of the onset, systolic peak, dicrotic notch, and diastolic peak within the third pulse are denoted as $A_{onset}''$, $A_{sp}''$, $A_{dn}''$ and $A_{dp}''$, respectively (i.e., with the addition of two prime symbols).

In an embodiment, upon receiving the PPG signals (e.g., analog electrical signals) output by the light sensor 125, the processing unit 120 may analyze the received PPG signals to identify respective pulses within the PPG signals. For example, a pulse within a PPG signal starts from the onset of a current pulse, and ends at the onset of the next pulse. For brevity and simplicity, the processing unit 120 is described as using the amplitudes of the feature points in the first pulse of the second PPG signal for subsequent calculations. It should be noted that the processing unit 120 may also use the amplitudes of the feature points in the respective pulse, which corresponds to the time interval of the first pulse in the second PPG signal, within each of the other PPG signals (such as those shown by curves 301, 303 to 30N) for subsequent calculations.

Specifically, the processing unit 120 identifies, from at least one pulse of each PPG signal, a respective set of fiducial points and a respective set of amplitude values associated with the respective set of fiducial points. In the scenario using two light sources, the processing unit 120 identifies, from at least one pulse of the first PPG signal (e.g., corresponding to the first wavelength band (WB1)), a first set of fiducial points (e.g., $A_{onset(WB1)}$, $A_{sp(WB1)}$, $A_{dn(WB1)}$, and $A_{dp(wB1)}$) and a first set of amplitude values associated with the first set of fiducial points. Then, the processing unit 120 identifies, from at least one pulse of the second PPG signal (e.g., corresponding to the second wavelength band (WB2)), a second set of fiducial points and a second set of amplitude values associated with the second set of fiducial points. Here, the first set of amplitude values includes the amplitudes $A_{sp(WB1)}$, $A_{dn(WB1)}$, and $A_{dp(WB1)}$ with reference to the amplitude $A_{onset(WB1)}$, while the second set of amplitude values includes the amplitudes $A_{sp(WB2)}$, $A_{dn(WB2)}$, and $A_{dp(WB2)}$ with reference to the amplitude $A_{onset(WB2)}$. For simplicity, the amplitudes $A_{sp(WB2)}$, $A_{dn(WB2)}$, and $A_{dp(WB2)}$ with reference to the amplitude $A_{onset(WB2)}$ in the selected pulse of the second PPG signal can be expressed as amplitudes $A1_{(WB2)}$, $A2_{(WB2)}$, and $A3_{(WB2)}$, respectively. Similarly, the amplitudes $A_{sp(WB1)}$, $A_{dn(WB1)}$, and $A_{dp(WB1)}$ with reference to the amplitude $A_{onset(WB1)}$ in the selected pulse of the first PPG signal can be expressed as amplitudes $A1_{(WB1)}$, $A2_{(WB1)}$, and $A3_{(WB1)}$, respectively. That is, $A1_{(WB1)} = A_{sp(WB1)} - A_{onset(WB1)}$, $A2_{(WB1)} = A_{dn(WB1)} - A_{onset(WB1)}$, and $A3_{(WB1)} = A_{dp(WB1)} - A_{onset(WB1)}$ for the first PPG signal corresponding to the first wavelength band. Similarly, $A1_{(WB2)} = A_{sp(WB2)} - A_{onset(WB2)}$, $A2_{(WB2)} = A_{dn(WB2)} - A_{onset(WB2)}$, and $A3_{(WB2)} = A_{dp(WB2)} - A_{onset(WB2)}$ for the second PPG signal corresponding to the second wavelength band.

In an embodiment, upon obtaining the first set of amplitude values and the second set of amplitude values from the first and second PPG signals, the processing unit 120 derives a plurality of amplitude ratios from the first and second sets of amplitude values. The plurality of amplitude ratios can be divided into a first subgroup G1 and a second subgroup G2. The first subgroup G1 includes amplitude ratios $R_{A1\_G1}$, $R_{A2\_G2}$, and $R_{A3\_G3}$, and the second subgroup G2 includes amplitude ratios $R_{A1\_G2}$, $R_{A2\_G2}$, and $R_{A3\_G2}$, which are defined as follows. (The subscript "_12" indicates that the PPG signals from the first and second wavelength bands are involved.)

$$R_{A1\_G1\_12} = \frac{A1_{(WB2)}}{A1_{(WB1)}} \qquad (1\text{-}1)$$

$$R_{A2\_G1\_12} = \frac{A2_{(WB2)}}{A2_{(WB1)}} \qquad (1\text{-}2)$$

$$R_{A3\_G1\_12} = \frac{A3_{(WB2)}}{A3_{(WB1)}} \qquad (1\text{-}3)$$

$$R_{A1\_G2\_12} = \frac{\log(A1_{(WB2)})}{\log(A1_{(WB1)})} - K \qquad (2\text{-}1)$$

$$R_{A2\_G2\_12} = \frac{\log(A2_{(WB2)})}{\log(A2_{(WB1)})} - K \qquad (2\text{-}2)$$

$$R_{A3\_G2\_12} = \frac{\log(A3_{(WB2)})}{\log(A3_{(WB1)})} - K \qquad (2\text{-}3)$$

More specifically, the amplitude ratio $R_{A1\_G1\_12}$ is derived by dividing the second systolic peak amplitude (e.g., $A1_{(WB2)}$) by the first systolic peak amplitude (e.g., $A1_{(WB1)}$). The amplitude ratio $R_{A2\_G1\_12}$ is derived by dividing the second dicrotic notch amplitude (e.g., $A2_{(WB2)}$) by the first dicrotic notch amplitude (e.g., $A2_{(WB1)}$). The amplitude ratio $R_{A3\_G1\_12}$ is derived by dividing the second diastolic peak amplitude (e.g., $A3_{(WB2)}$) by the first diastolic peak amplitude (e.g., $A3_{(WB1)}$).

Additionally, the amplitude ratio $R_{A1\_G2\_12}$ is derived by dividing the logarithm of the second systolic peak amplitude (e.g., $\log(A1_{(WB2)})$) by the logarithm of the first systolic peak amplitude (e.g., $\log(A1_{(WB1)})$), and optionally subtracted by a constant K, which is a constant in a regression curve. The amplitude ratio $R_{A2\_G2\_12}$ is derived by dividing the logarithm of the second dicrotic notch amplitude (e.g., $\log(A2_{(WB2)})$) by the logarithm of the first dicrotic notch amplitude (e.g., $\log(A2_{(WB1)})$), and optionally subtracted by a constant K. The amplitude ratio $R_{A3\_G2\_12}$ is derived by dividing the logarithm of the second diastolic peak amplitude (e.g., $\log(A3_{(WB2)})$) by the logarithm of the first diastolic peak amplitude (e.g., $\log(A3_{(WB1)})$), and optionally subtracted by a constant K.

In an embodiment, upon deriving the six amplitude ratios (e.g., $R_{A1\_G1\_12}$, $R_{A2\_G1\_12}$, $R_{A3\_G1\_12}$, $R_{A1\_G2\_12}$, $R_{A2\_G2\_12}$, and $R_{A3\_G2\_12}$) noted above, the processing unit 120 is further configured to apply the six amplitude ratios into a regression model. As such, the regression model is capable of estimating the blood glucose level (e.g., blood glucose concentration) of the user. The regression model may include linear regression, support vector regression, random forest regression, and the like, but the present disclosure is not limited thereto. Additionally, the regression model may be established and trained using large datasets, which correspond to at least two wavelength bands, collected from different users. The amplitudes of fiducial points within each PPG signal in a respective wavelength band and collected blood glucose levels may serve as inputs during the training stage of the regression model, enabling the regression model to estimate the blood glucose level of the user. For example, when a user wears a wearable device including the apparatus 10, the apparatus 10 can generate the PPG signals detected from lights in different wavelengths or wavelength bands to calculate amplitudes of fiducial points of a selected pulse in each PPG signal. Then, the processing unit 120 can calculate the amplitude ratios (e.g., $R_{A1\_G1\_12}$, $R_{A2\_G1\_12}$, $R_{A3\_G1\_12}$, $R_{A1\_G2\_12}$, $R_{A2\_G2\_12}$, and $R_{A3\_G2\_12}$), and apply the amplitude ratios to the regression model to estimate the blood glucose level of the user.

It is possible that not all six amplitude ratios noted above are used. In an embodiment, the processing unit 120 applies at least one of the calculated amplitude ratios (e.g., $R_{A1\_G1\_12}$, $R_{A2\_G1\_12}$, $R_{A3\_G1\_12}$, $R_{A1\_G2\_12}$, $R_{A2\_G2\_12}$, and $R_{A3\_G2\_12}$) to the regression model to estimate the blood glucose level of the user. In an embodiment, upon obtaining the first set of amplitude values and the second set of amplitude values, the processing unit 120 derives a plurality of amplitude ratios from the first and second sets of amplitude values. The plurality of amplitude ratios includes at least one of the amplitude ratios from the first subgroup G1, such as $R_{A1\_G1\_12}$, $R_{A2\_G1\_12}$, and $R_{A3\_G1\_12}$. Then, the processing unit 120 inputs the at least one calculated amplitude ratio in the first subgroup G1 to the regression model to estimate the blood glucose level of the user. Optionally, to lower the computation complexity, the processing unit 120 derives the amplitude ratio $R_{A1\_G1\_12}$ from the first and second sets of amplitude values, and inputs the amplitude ratio $R_{A1\_G1\_12}$ to the regression model to estimate the blood glucose level of the user.

In an embodiment, upon obtaining the first set of amplitude values and the second set of amplitude values, the processing unit 120 derives a plurality of amplitude ratios from the first and second sets of amplitude values. The plurality of amplitude ratios includes at least one of the amplitude ratios from the second subgroup G2, such as $R_{A1\_G2\_12}$, $R_{A2\_G2\_12}$, and $R_{A3\_G2\_12}$. Then, the processing unit 120 inputs the at least one calculated amplitude ratio in the second subgroup G2 to the regression model to estimate the blood glucose level of the user. Optionally, to lower the computation complexity, the processing unit 120 derives the amplitude ratio $R_{A1\_G2\_12}$ from the first and second sets of amplitude values, and inputs the amplitude ratio $R_{A1\_G2\_12}$ to the regression model to estimate the blood glucose level of the user.

In an embodiment, upon obtaining the first set of amplitude values and the second set of amplitude values, the processing unit 120 derives a plurality of amplitude ratios from the first and second sets of amplitude values. The plurality of amplitude ratios includes amplitude ratios $R_{A1\_G1\_12}$ and $R_{A1\_G2\_12}$. Then, the processing unit 120 inputs the calculated amplitude ratios $R_{A1\_G1\_12}$ and $R_{A1\_G2\_12}$ to the regression model to estimate the blood glucose level of the user.

In the scenario using three light sources, the processing unit 120 is further configured to identify, from at least one pulse of the third PPG signal (e.g., corresponding to the third wavelength band (WB3)), a third set of fiducial points (e.g., $A_{onset(WB3)}$, $A_{sp(WB3)}$, $A_{dn(WB3)}$, and $A_{dp(WB3)}$) and a third set of amplitude values associated with the third set of fiducial points. Here, the third set of amplitude values includes the amplitudes $A_{sp(WB3)}$, $A_{dn(WB3)}$, and $A_{dp(WB3)}$ with reference to the amplitude $A_{onset(WB3)}$, which can be expressed as amplitudes $A1_{(WB3)}$, $A2_{(WB3)}$, and $A3_{(WB3)}$, respectively. That is, $A1_{(WB3)} = A_{sp(WB3)} - A_{onset(WB3)}$, $A2_{(WB3)} = A_{dn(WB3)} - A_{onset(WB3)}$, and $A3_{(WB3)} = A_{dp(WB3)} - A_{onset(WB3)}$ for the third PPG signal corresponding to the third wavelength band.

Upon obtaining the first to third sets of amplitude values from the first to third PPG signals, the processing unit 120 is configured to derive a plurality of amplitude ratios from the first to third sets of amplitude values. In addition to the first subgroup G1 and second subgroup G2 derived from the first and second sets of amplitude values, the processing unit 120 is further configured to derive a first subgroup G1 and a second subgroup G2 from the first and third sets of amplitude values, which are defined as follows. (The subscript "_13" indicates that the PPG signals from the first and third wavelength bands are involved.)

$$R_{A1\_G1\_13} = \frac{A1_{(WB3)}}{A1_{(WB1)}} \tag{3-1}$$

$$R_{A2\_G1\_13} = \frac{A2_{(WB3)}}{A2_{(WB1)}} \tag{3-2}$$

$$R_{A3\_G1\_13} = \frac{A3_{(WB3)}}{A3_{(WB1)}} \tag{3-3}$$

$$R_{A1\_G2\_13} = \frac{\log(A1_{(WB3)})}{\log(A1_{(WB1)})} - K \tag{4-1}$$

$$R_{A2\_G2\_13} = \frac{\log(A2_{(WB3)})}{\log(A2_{(WB1)})} - K \tag{4-2}$$

$$R_{A3\_G2\_13} = \frac{\log(A3_{(WB3)})}{\log(A3_{(WB1)})} - K \tag{4-3}$$

More specifically, the amplitude ratio $R_{A1\_G1\_13}$ is derived by dividing the third systolic peak amplitude (e.g., $A1_{(WB3)}$) by the first systolic peak amplitude (e.g., $A1_{(wB1)}$). The amplitude ratio $R_{A2\_G1\_13}$ is derived by dividing the third dicrotic notch amplitude (e.g., $A2_{(WB3)}$) by the first dicrotic notch amplitude (e.g., $A2_{(WB1)}$). The amplitude ratio $R_{A3\_G1\_13}$ is derived by dividing the third diastolic peak amplitude (e.g., $A3_{(WB3)}$) by the first diastolic peak amplitude (e.g., $A3_{(WB1)}$).

Additionally, the amplitude ratio $R_{A1\_G2\_13}$ is derived by dividing the logarithm of the third systolic peak amplitude (e.g., $\log(A1_{(WB3)})$) by the logarithm of the first systolic peak amplitude (e.g., $\log(A1_{(WB1)})$), and optionally subtracted by a constant K (a constant in the regression curve). The amplitude ratio $R_{A2\_G2\_13}$ is derived by dividing the logarithm of the third dicrotic notch amplitude (e.g., $\log(A2_{(WB3)})$) by the logarithm of the first dicrotic notch amplitude (e.g., $\log(A2_{(WB1)})$), and optionally subtracted by a constant K. The amplitude ratio $R_{A3\_G2\_13}$ is derived by dividing the logarithm of the third diastolic peak amplitude (e.g., $\log(A3_{(WB3)})$) by the logarithm of the first diastolic peak amplitude (e.g., $\log(A3_{(WB1)})$), and optionally subtracted by a constant K.

In an embodiment, upon deriving the twelve amplitude ratios using equations 1-1 to 1-13, 2-1 to 2-3, 3-1 to 3-3, and 4-1 to 4-3, the processing unit 120 is further configured to apply the twelve amplitude ratios into a regression model. As such, the regression model is capable of estimating the blood glucose level (e.g., blood glucose concentration) of the user.

In an embodiment, upon obtaining the first to third sets of amplitude values, the processing unit 120 derives a plurality of amplitude ratios from the first to third sets of amplitude values. The plurality of amplitude ratios includes at least one of amplitude ratio in the first subgroup G1, such as $R_{A1\_G1\_12}$, $R_{A2\_G1\_12}$, and $R_{A3\_G1\_12}$. Then, the processing unit 120 inputs the at least one calculated amplitude ratio in the first subgroup G1 to the regression model to estimate the blood glucose level of the user.

Optionally, to lower the computation complexity, the processing unit 120 derives the amplitude ratio $R_{A1\_G1\_12}$

from the first and second sets of amplitude values, and inputs the amplitude ratio $R_{A1\_G1\_12}$ to the regression model to estimate the blood glucose level of the user. Optionally, the processing unit 120 derives the amplitude ratio $R_{A1\_G1\_13}$ from the first and third sets of amplitude values, and inputs the amplitude ratio $R_{A1\_G1\_13}$ to the regression model to estimate the blood glucose level of the user.

In an embodiment, the processing unit 120 derives at least one first amplitude ratio from the first and second sets of amplitudes, and at least one second amplitude ratio from the first and third sets of amplitudes. The processing units 120 inputs the at least one first amplitude ratio and the at least one second amplitude ratio to the regression model to estimate the blood glucose level of the user. For example, the at least one first amplitude ratio includes the amplitude ratios $R_{A1\_G1\_12}$ and $R_{A1\_G2\_12}$, and the at least one second amplitude ratio includes the amplitude ratios $R_{A1\_G1\_13}$ and $R_{A1\_G2\_13}$. Optionally, the at least one first amplitude ratio includes the amplitude ratios $R_{A1\_G1\_12}$, and the at least one second amplitude ratio includes the amplitude ratios $R_{A1\_G1\_13}$.

In the scenario using N light sources (N > 3), the processing unit 120 is configured to identify, from at least one pulse of the respective PPG signal corresponding to each wavelength band (e.g., WBn, where n is a positive integer between 1 and N), a n-th set of fiducial points (e.g., $A_{onset(WBn)}$, $A_{sp(WBn)}$, $A_{dn(WBn)}$, and $A_{dp(WBn)}$) and a n-th set of amplitude values associated with the n-th set of fiducial points. Here, the n-th set of amplitude values includes the amplitudes $A_{sp(WBn)}$, $A_{dn(WBn)}$, and $A_{dp(WBn)}$ with reference to the amplitude $A_{onset(WBn)}$, which can be expressed as amplitudes $A1_{(WBn)}$, $A2_{(WBn)}$, and $A3_{(WBn)}$, respectively. That is, $A1_{(WBn)} = A_{sp(WBn)} - A_{onset(WBn)}$, $A2_{(WBn)}=A_{dn(WBn)} - A_{onset(WBn)}$, and $A3_{(WBn)} = A_{dp(WBn)} - A_{onset(WBn)}$ for the third PPG signal corresponding to the third wavelength band.

Upon obtaining the first to N-th sets of amplitude values from the first to N-th PPG signals, the processing unit 120 is configured to derive a plurality of amplitude ratios from the first to third sets of amplitude values. The processing unit 120 is further configured to derive a first subgroup G1 and a second subgroup G2 from the first and n-th (e.g., n is a positive integer between 2 and N) sets of amplitude values, which are defined as follows. (The subscript "_1n" indicates that the PPG signals from the first and n-th wavelength bands are involved.)

$$R_{A1\_G1\_1n} = \frac{A1_{(WBn)}}{A1_{(WB1)}} \qquad (5\text{-}1)$$

$$R_{A2\_G1\_1n} = \frac{A2_{(WBn)}}{A2_{(WB1)}} \qquad (5\text{-}2)$$

$$R_{A3\_G1\_1n} = \frac{A3_{(WBn)}}{A3_{(WB1)}} \qquad (5\text{-}3)$$

$$R_{A1\_G2\_1n} = \frac{\log\left(A1_{(WBn)}\right)}{\log\left(A1_{(WB1)}\right)} - K \qquad (6\text{-}1)$$

$$R_{A2\_G2\_1n} = \frac{\log\left(A2_{(WBn)}\right)}{\log\left(A2_{(WB1)}\right)} - K \qquad (6\text{-}2)$$

$$R_{A3\_G2\_1n} = \frac{\log\left(A3_{(WBn)}\right)}{\log\left(A3_{(WB1)}\right)} - K \qquad (6\text{-}3)$$

More specifically, the amplitude ratio $R_{A1\_G1\_1n}$ is derived by dividing the n-th systolic peak amplitude (e.g., $A1_{(WBn)}$) by the first systolic peak amplitude (e.g., $A1_{(WB1)}$). The amplitude ratio $R_{A2\_G1\_1n}$ is derived by dividing the n-th dicrotic notch amplitude (e.g., $A2_{(WBn)}$) by the first dicrotic notch amplitude (e.g., $A2_{(WB1)}$). The amplitude ratio $R_{A3\_G1\_1n}$ is derived by dividing the n-th diastolic peak amplitude (e.g., $A3_{(WBn)}$) by the first diastolic peak amplitude (e.g., $A3_{(WB1)}$).

Additionally, the amplitude ratio $R_{A1\_G2\_1n}$ is derived by dividing the logarithm of the n-th systolic peak amplitude (e.g., $\log(A1_{(WBn)})$) by the logarithm of the first systolic peak amplitude (e.g., $\log(A1_{(WB1)})$), and optionally subtracted by a constant K (a constant in the regression curve). The amplitude ratio $R_{A2\_G2\_1n}$ is derived by dividing the logarithm of the n-th dicrotic notch amplitude (e.g., $\log(A2_{(WBn)})$) by the logarithm of the first dicrotic notch amplitude (e.g., $\log(A2_{(WB1)})$), and optionally subtracted by a constant K. The amplitude ratio $R_{A3\_G2\_1n}$ is derived by dividing the logarithm of the n-th diastolic peak amplitude (e.g., $\log(A3_{(WBn)})$) by the logarithm of the first diastolic peak amplitude (e.g., $\log(A3_{(WB1)})$), and optionally subtracted by a constant K.

In an embodiment, upon deriving the amplitude ratios within the first subgroup G1 and second subgroup G2 for every value of n between 2 and N, the processing unit 120 is further configured to apply all the derived amplitude ratios into a regression model. As such, the regression model is capable of estimating the blood glucose level (e.g., blood glucose concentration) of the user.

In an embodiment, upon obtaining the first to N-th sets of amplitude values, the processing unit 120 derives a plurality of amplitude ratios from the first to N-th sets of amplitude values. The plurality of amplitude ratios includes at least one of the amplitude ratios from the first subgroup G1, such as $R_{A1\_G1\_1N}$, $R_{A2\_G1\_1N}$, and $R_{A3\_G1\_1N}$. Then, the processing unit 120 inputs the at least one calculated amplitude ratio in the first subgroup G1 to the regression model to estimate the blood glucose level of the user.

Optionally, to lower the computation complexity, the processing unit 120 derives the amplitude ratio $R_{A1\_G1\_1n}$ from the first and n-th sets of amplitude values, and inputs the amplitude ratio $R_{A1\_G1\_1n}$ to the regression model to estimate the blood glucose level of the user, where n is a positive integer between 2 and N. In other words, for any given value of n, the amplitude ratio $R_{A1\_G1\_1n}$ of the systolic peak amplitude $A1_{(WBn)}$ associated with the n-th wavelength band to the systolic peak amplitude $A1_{(WB1)}$ associated with the first wavelength band can be input to the regression model to estimate the blood glucose level of the user.

In an embodiment, the processing unit 120 derives at least one amplitude ratio from the first and n-th (e.g., for every n between 2 and N) sets of amplitude values. The processing units 120 inputs the at least one amplitude ratio, which is derived from the first and n-th sets of amplitude values, to the regression model to estimate the blood glucose level of the user. For example, the at least one amplitude ratio, which is derived from the first and n-th sets of amplitude values, includes the amplitude ratios $R_{A1\_G1\_12}$, $R_{A1\_G2\_12}$, $R_{A1\_G1\_13}$, $R_{A1\_G2\_13}$, ...., $R_{A1\_G1\_1N}$, and $R_{A1\_G2\_1N}$. In other words, the amplitude ratios associated with the systolic peak amplitude in the first subgroup G1 and the second subgroup G2 for each combination of the first and n-th wavelength bands are input to the regression model to estimate the blood glucose level of the user. Optionally, the at least one amplitude ratio, which is derived from the first and second sets of amplitude values, includes the amplitude ratios $R_{A1\_G1\_12}$, $R_{A1\_G1\_13}$, ..., and $R_{A1\_G1\_1N}$. In other words, the amplitude ratios associated with the systolic peak amplitude in the first subgroup G1 for each combination of the first and n-th wavelength bands are input to the regression model to estimate the blood glucose level of the user.

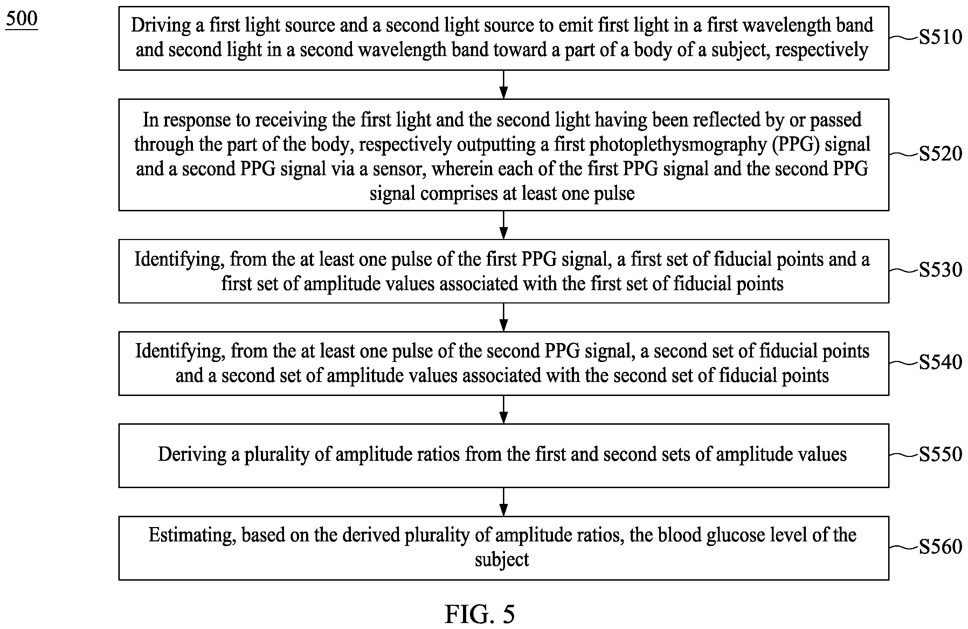

FIG. 5 is a flowchart of a method for non-invasive measurement of blood glucose level in accordance with an embodiment of the present disclosure. Please refer to both FIGS. 1 and 5. The method 500 includes steps S510 to S560.

In step S510, a first light source and a second light source are driven to emit first light in a first wavelength band and second light in a second wavelength band toward a part of a body of a subject, respectively. For example, the arrangement of the first light source and second light source can be referred to the embodiments and scenarios using two light sources described above. Additionally, glucose has lower absorption level in the first wavelength band than the second wavelength band, and hemoglobin has higher absorption level in the first wavelength band than in the second wavelength band.

In step S520, in response to receiving the first light and the second light having been reflected by or passed through the part of the body, respectively outputting a first photoplethysmography (PPG) signal and a second PPG signal via a sensor, wherein each of the first PPG signal and the second PPG signal comprises at least one pulse. For example, the first light source and the second light source are disposed at the same side of the part of the body, and the sensor (e.g., light sensor 125) may be disposed at the same side of the first light source and the second light source to receive the first light and the second light having been reflected by the part of the body, or disposed at an opposite side of the first light source and the second light source to receive the first light and the second light having been passed through the part of the body.

In step S530, a first set of fiducial points and a first set of amplitudes values associated with the first set of fiducial points are identified from the at least one pulse of the first PPG signal. For example, the first set of fiducial points includes an onset, systolic peak, dicrotic notch, and diastolic peak within the at least one pulse of the first PPG signal. The first set of amplitude values includes the systolic peak amplitude, dicrotic notch amplitude, and diastolic peak amplitude with reference to the onset amplitude within the at least one pulse of the first PPG signal.

In step S540, a second set of fiducial points and a second set of amplitudes values associated with the second set of fiducial points are identified from the at least one pulse of the second PPG signal. For example, the second set of fiducial points includes an onset, systolic peak, dicrotic notch, and diastolic peak within the at least one pulse of the second PPG signal. The second set of amplitude values includes the systolic peak amplitude, dicrotic notch amplitude, and diastolic peak amplitude with reference to the onset amplitude within the at least one pulse of the second PPG signal. It should be noted that the order of steps S530 and S540 is interchangeable.

In step S550, a plurality of amplitude ratios are derived from the first and second sets of amplitude values. In an embodiment, the plurality of amplitude ratios may include the amplitude ratios $R_{A1\_G1\_12}$, $R_{A2\_G1\_12}$, $R_{A3\_G1\_12}$, $R_{A1\_G2\_12}$, $R_{A2\_G2\_12}$, and $R_{A3\_G2\_12}$ as noted. Optionally, the plurality of amplitude ratios includes at least one of the amplitude ratios from the first subgroup G1, such as $R_{A1\_G1\_12}$, $R_{A2\_G1\_12}$, and $R_{A3\_G1\_12}$. Optionally, the plurality of amplitude ratios includes at least one of the amplitude ratios from the second subgroup G2, such as $R_{A1\_G2\_12}$, $R_{A2\_G2\_12}$, and $R_{A3\_G2\_12}$. Optionally, the plurality of amplitude ratios includes amplitude ratios $R_{A1\_G1\_12}$ and $R_{A1\_G2\_12}$.

In step S560, the blood glucose level of the subject is estimated based on the derived plurality of amplitude ratios. In an embodiment, the processing unit 120 can input the derived plurality of amplitude ratios, which corresponds to one of possible combinations described in step S550, into a regression model to estimate the blood glucose level of the subject.

Accordingly, an apparatus and a method for non-invasive measurement of blood glucose level are provided, which are capable of driving two or more light sources to emit lights in different wavelength bands toward a part of a body of a user (e.g., a subject), deriving amplitude values from fiducial points in at least one pulse of a respective PPG signal corresponding to each wavelength band, and estimating the blood glucose level of the user using a plurality of amplitude ratios derived from the amplitude values corresponding to different combinations of wavelength bands. The lights emitted by the two or more light sources are in different wavelength bands, indicating that the lights have different characteristics of absorption coefficients for at least glucose and hemoglobin. This helps build the regression model using more data points collected for cross-referencing and calibration compared to using one light source, thereby improving overall accuracy in the estimation of the blood glucose level of the subject.

The above is only exemplary, rather than restrictive. Any equivalent modifications or changes without departing from the scope of the present disclosure should fall within the scope of the appended claims.

**Claims**

1. An apparatus for non-invasive measurement of a blood glucose level of a subject, the apparatus comprising:

   a first light source configured to emit first light in a first wavelength band toward a part of a body of the subject;
   a second light source configured to emit second light in a second wavelength band toward the part of the body;
   a sensor configured to, in response to receiving the first light and the second light having been reflected by or passed through the part of the body, respectively output a first photoplethysmography (PPG) signal and a second PPG signal, each of the first PPG signal and the second PPG signal comprising at least one pulse; and
   a processor configured to:

      identify, from the at least one pulse of the first PPG signal, a first set of fiducial points and a first set of amplitude values associated with the first set of fiducial points;
      identify, from the at least one pulse of the second PPG signal, a second set of fiducial points and a second set of amplitude values associated with the second set of fiducial points;
      deriving a plurality of amplitude ratios from the first and second sets of amplitude values; and
      estimating, based on the derived plurality of amplitude ratios, the blood glucose level of the subject;

   wherein glucose has lower absorption level in the first wavelength band than the second wavelength band,
   wherein hemoglobin has higher absorption level in the first wavelength band than in the second wavelength band.

2. The apparatus according to Claim 1, wherein:

   the first set of fiducial points comprises a first valley, a first systolic peak, a first dicrotic notch and a first diastolic peak;
   the second set of fiducial points comprises a second valley, a second systolic peak, a second dicrotic notch and a second diastolic peak;
   the first set of amplitude values comprises a first systolic peak amplitude, a first dicrotic notch amplitude, and a first diastolic peak amplitude each measured with reference to a first valley amplitude; and
   the second set of amplitude values comprises a second systolic peak amplitude, a second dicrotic notch amplitude, and a second diastolic peak amplitude each measured with reference with a second valley amplitude.

3. The apparatus according to any one of Claims 1 to 2, wherein the plurality of amplitude ratios comprise at least one of:

   the second systolic peak amplitude divided by the first systolic peak amplitude;
   the second dicrotic notch amplitude divided by the first dicrotic notch amplitude;

the second diastolic peak amplitude divided by the first diastolic peak amplitude;

a logarithm of the second systolic peak amplitude divided by a logarithm of the first systolic peak amplitude, and optionally subtracted by a constant;

a logarithm of the second dicrotic notch amplitude divided by a logarithm of the first dicrotic notch amplitude, and optionally subtracted by a constant; and

a logarithm of the second diastolic peak amplitude divided by a logarithm of the first diastolic peak amplitude, and optionally subtracted by a constant.

4.  The apparatus according to any one of Claims 1 to 3, wherein the processor is further configured to apply the plurality of amplitude ratios into a regression model, and estimate the blood glucose level of the subject based on the regression model.

5.  The apparatus according to any one of Claims 1 to 4, wherein:

the absorption level of glucose in the first wavelength band is less than 0.003 $cm^{-1} \cdot (mol/L)^{-1}$, and/or the absorption levels for both oxyhemoglobin (HbO2) and deoxyhemoglobin (Hb) in the first wavelength band are more than 10 $cm^{-1} \cdot (mol/L)^{-1}$, in which the $L$ denotes liter ($cm^3$) and $mol$ is the unit of mole;

oxyhemoglobin (HbO2) and deoxyhemoglobin (Hb) have substantially identical absorption level in the first wavelength band, optionally the absorbance coefficient for both Hb and HbO2 are around 10 $cm^{-1} \cdot (mol/L)^{-1}$, in which the L denotes liter ($cm^3$) and mol is the unit of mole; and/or

the first wavelength band is green light, optionally around 520nm.

6.  The apparatus according to any one of Claims 1 to 5, wherein:

the absorption level of glucose in the second wavelength band is more than 0.005 $cm^{-1} \cdot (mol/L)^{-1}$, and/or the absorption levels of hemoglobin in the second wavelength band for both Hb and HbO2 are less than 10 $cm^{-1} \cdot (mol/L)^{-1}$; and/or

the second wavelength band is near-infrared (NIR) light, optionally around 940nm.

7.  The apparatus according to any one of Claims 1 to 6, further comprising a third light source configured to emit third light in a third wavelength band, towards the part of the body of the subject, wherein:

the absorption level of hemoglobin in the third wavelength band is greater than 0.2 $cm^{-1} \cdot (mol/L)^{-1}$;

glucose has higher absorption level in the third wavelength band than the second wavelength band, optionally the absorption level of glucose in the third wavelength band is greater than 0.2 $cm^{-1} \cdot (mol/L)^{-1}$; and/or

the third wavelength band is infrared light, optionally around 1550nm.

8.  The apparatus according to Claim 7, wherein:

the sensor is further configured to, in response to receiving the third light having been reflected by or passed through the part of the body, output a third PPG signal, the third PPG signal comprising at least one pulse;

the processor is further configured to identify, from the at least one pulse of the third PPG signal, a third set of fiducial points and a third set of amplitude values associated with the third set of fiducial points;

the third set of fiducial points comprises a third valley, a third systolic peak, a third dicrotic notch and a third diastolic peak;

the plurality of amplitude ratios are further derived from the third set of amplitude values; and

the third set of amplitude values comprises a third systolic peak amplitude, a third dicrotic notch amplitude and a third diastolic peak amplitude each measured with reference to a third valley amplitude.

9.  The apparatus according to any one of Claims 7 and 8, wherein the plurality of amplitude ratios further comprise at least one of:

the third systolic peak amplitude divided by the first systolic peak amplitude;

the third dicrotic notch amplitude divided by the first dicrotic notch amplitude;

the third diastolic peak amplitude divided by the first diastolic peak amplitude;

a logarithm of the third systolic peak amplitude divided by a logarithm of the first systolic peak amplitude, and optionally subtracted by a constant;

a logarithm of the third dicrotic notch amplitude divided by a logarithm of the first dicrotic notch amplitude, and optionally subtracted by a constant; and

a logarithm of the third diastolic peak amplitude divided by a logarithm of the first diastolic peak amplitude, and optionally subtracted by a constant.

10. A method for estimating a blood glucose level of a subject using non-invasive measurement, the method comprising:

driving a first light source and a second light source to emit first light in a first wavelength band and second light in a second wavelength band toward a part of a body of a subject, respectively;

in response to receiving the first light and the second light having been reflected by or passed through the part of the body, respectively outputting a first photoplethysmography (PPG) signal and a second PPG signal via a sensor, wherein each of the first PPG signal and the second PPG signal comprises at least one pulse;

identifying, from the at least one pulse of the first PPG signal, a first set of fiducial points and a first set of amplitude value s associated with the first set of fiducial points;

identifying, from the at least one pulse of the second PPG signal, a second set of fiducial points and a second set of amplitude values associated with the second set of fiducial points;

deriving a plurality of amplitude ratios from the first and second sets of amplitude values; and

estimating, based on the derived plurality of amplitude ratios, the blood glucose level of the subject,

wherein glucose has lower absorption level in the first wavelength band than the second wavelength band,

wherein hemoglobin has higher absorption level in the first wavelength band than in the second wavelength band.

11. The method according to Claim 10, wherein:

the first set of fiducial points comprises a first valley, a first systolic peak, a first dicrotic notch and a first diastolic peak;

the second set of fiducial points comprises a second valley, a second systolic peak, a second dicrotic notch and a second diastolic peak;

the first set of amplitude values comprises a first systolic peak amplitude, a first dicrotic notch amplitude, and a first diastolic peak amplitude each measured with reference to a first valley amplitude; and

the second set of amplitude values comprises a second systolic peak amplitude, a second dicrotic notch amplitude, and a second diastolic peak amplitude each measured with reference with a second valley amplitude.

12. The method according to any one of Claims 10 and 11, wherein the plurality of amplitude ratios comprise at least one of:

the second systolic peak amplitude divided by the first systolic peak amplitude;

the second dicrotic notch amplitude divided by the first dicrotic notch amplitude;

the second diastolic peak amplitude divided by the first diastolic peak amplitude;

a logarithm of the second systolic peak amplitude divided by a logarithm of the first systolic peak amplitude, and optionally subtracted by a constant;

a logarithm of the second dicrotic notch amplitude divided by a logarithm of the first dicrotic notch amplitude, and optionally subtracted by a constant; and

a logarithm of the second diastolic peak amplitude divided by a logarithm of the first diastolic peak amplitude, and optionally subtracted by a constant.

13. The method according to any one of Claims 10 to 12, further comprising: apply the plurality of amplitude ratios into a regression model, and estimating the blood glucose level of the subject based on the regression model.

14. The method according to any one of Claims 10 to 13, wherein:

the absorption level of glucose in the first wavelength band is less than 0.003 $cm^{-1} \cdot (mol/L)^{-1}$, and/or the absorption levels for both oxyhemoglobin (HbO2) and deoxyhemoglobin (Hb) in the first wavelength band are more than 10 $cm^{-1} \cdot (mol/L)^{-1}$, in which the L denotes liter ($cm^3$) and mol is the unit of mole;

oxyhemoglobin (HbO2) and deoxyhemoglobin (Hb) have substantially identical absorption level in the first wavelength band, optionally the absorbance coefficient for both Hb and HbO2 are around 10 $cm^{-1} \cdot (mol/L)^{-1}$, in which the L denotes liter ($cm^3$) and mol is the unit of mole; and/or

the first wavelength band is green light, optionally around 520nm..

15. The method according to any one of Claims 10 to 14, wherein:

the absorption level of glucose in the second wavelength band is more than 0.005 $cm^{-1} \cdot (mol/L)^{-1}$, and/or the absorption levels of hemoglobin in the second wavelength band for both Hb and HbO2 are less than 10 $cm^{-1} \cdot (mol/L)^{-1}$; and/or
the second wavelength band is near-infrared (NIR) light, optionally around 940nm.

FIG. 1

EP 4 699 534 A1

FIG. 2

EP 4 699 534 A1

FIG. 3

EP 4 699 534 A1

FIG. 4

500

| Driving a first light source and a second light source to emit first light in a first wavelength band and second light in a second wavelength band toward a part of a body of a subject, respectively | ~S510 |

In response to receiving the first light and the second light having been reflected by or passed through the part of the body, respectively outputting a first photoplethysmography (PPG) signal and a second PPG signal via a sensor, wherein each of the first PPG signal and the second PPG signal comprises at least one pulse — S520

Identifying, from the at least one pulse of the first PPG signal, a first set of fiducial points and a first set of amplitude values associated with the first set of fiducial points — S530

Identifying, from the at least one pulse of the second PPG signal, a second set of fiducial points and a second set of amplitude values associated with the second set of fiducial points — S540

Deriving a plurality of amplitude ratios from the first and second sets of amplitude values — S550

Estimating, based on the derived plurality of amplitude ratios, the blood glucose level of the subject — S560

FIG. 5

EP 4 699 534 A1

Europäisches
Patentamt

European
Patent Office

Office européen
des brevets

# EUROPEAN SEARCH REPORT

**Application Number**

EP 25 19 7423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEE PO-LEI ET AL: "A Noninvasive Blood Glucose Estimation System Using Dual-Channel PPGs and Pulse-Arrival Velocity", IEEE SENSORS JOURNAL, IEEE, vol. 23, no. 19, 23 August 2023 (2023-08-23), pages 23570-23582, XP011950617, ISSN: 1530-437X, DOI: 10.1109/JSEN.2023.3306343 [retrieved on 2023-08-23] * abstract * * tables II, III * * page 23570 - page 23581 * ----- | 1-15 | INV. A61B5/145 A61B5/1455 |
| A | US 2021/068717 A1 (KIM DOUG WON [KR]) 11 March 2021 (2021-03-11) * paragraphs [0022] - [0036] * ----- | 1-15 | |
| A | RAY DANIEL ET AL: "A Review of Wearable Multi-Wavelength Photoplethysmography", IEEE REVIEWS IN BIOMEDICAL ENGINEERING, IEEE, USA, vol. 16, 19 October 2021 (2021-10-19), pages 136-151, XP011932049, ISSN: 1937-3333, DOI: 10.1109/RBME.2021.3121476 [retrieved on 2021-10-20] * page 136 - page 148 * ----- | 1-15 | TECHNICAL FIELDS SEARCHED (IPC) A61B |

The present search report has been drawn up for all claims

| Place of search | Date of completion of the search | Examiner |
|---|---|---|
| The Hague | 1 October 2025 | Athanasiadis, I |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.............................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 19 7423

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2025

| Patent document cited in search report | Publication date | Patent family member(s) | | Publication date |
|---|---|---|---|---|
| US 2021068717 A1 | 11-03-2021 | EP | 3763291 A1 | 13-01-2021 |
| | | KR | 20190105422 A | 17-09-2019 |
| | | US | 2021068717 A1 | 11-03-2021 |
| | | WO | 2019172570 A1 | 12-09-2019 |

EPO FORM P0459

For more details about this annex : see Official Journal of the European Patent Office, No. 12/82